# EUROPEAN PATENT APPLICATION

(11) **EP 2 233 557 A1**
(43) Date of publication of application: **29.09.2010**
(21) Application number: 09156355.1
(22) Date of filing: 26.03.2009
(51) Int. Cl.: C11D 3/50, B01J 13/18, B01J 13/12

(54) **A perfume encapsulate, a laundry detergent composition comprising a perfume encapsulate, and a process for preparing a perfume encapsulate**

(71) Applicant: The Procter and Gamble Company, Cincinnati, Ohio 45202 (US)
(72) Inventor: Blyth, Kevin Graham, Whitley Bay, Tyne and Wear NE26 4NU (GB); Meli, Fabrizio, North Shields, Tyne and Wear NE30 4DH (GB)
(74) Representative: Howard, Phillip Jan

(57) **Abstract**

The present invention relates to a process for preparing an encapsulate, the process comprises the steps of: (i) emulsifying a hydrophobic perfume-polymer complex in water to form an oil in water emulsion; (ii) at least partially dissolving an encapsulating material in water; (iii) removing at least some water from the oil in water emulsion comprising the hydrophobic perfume-polymer complex and the encapsulating material to form an encapsulate.

## Description

### FIELD OF THE INVENTION

The present invention relates to a process for preparing a perfume encapsulate. The perfume encapsulates are suitable for use in laundry detergent compositions. The perfume encapsulates exhibit improved thermal stability, and are capable of comprising high loads of perfume raw materials.

### BACKGROUND OF THE INVENTION

Current processing of a cohesive, perfume/polymer complex, such as the agglomeration process described in WO00/02986, uses a lot of carrier and coating materials and results in a relatively low activity perfume particle. In addition, the resultant perfume particles have poor thermal stability during storage/processing, having a tendency to become sticky and cake, especially in hot and humid countries such as Egypt and Saudi Arabia and the like.

The Inventors have overcome this problem by using an emulsification and encapsulation process, wherein a hydrophobic perfume-polymer complex is emulsified in water, an encapsulating material is at least partially dissolved in water (either before or after the emulsification step), and the mixture is dried to form an encapsulate.

The encapsulates produced by this process exhibit improved thermal stability, and can achieve higher loading levels of perfume raw materials.

In addition, the prior art particles (agglomerates) such as those described in WO00/02986 are relatively large; because a lot of binder and coating material is needed to form the agglomerates. This leads to poor dissolution profile.

The process of the present invention overcomes this problem by being able to make relatively small perfume encapsulate particles that exhibit good dissolution profiles, whilst retaining good thermal stability profiles. The encapsulates preferably have a weight average particle size of from 10 micrometers to 300 micrometers, preferably from 10 micrometers to 200 micrometers, more preferably from 40 micrometers to 120 micrometers.

In another embodiment of the present invention, preferably the encapsulating material does not melt at 135°C or less, preferably 170°C or less. This further improves the thermal stability of the encapsulate.

In another embodiment of the present invention, preferably the encapsulating material is thermally stable at 135°C or less, preferably 170°C or less. This further improves the thermal stability of the encapsulate.

In another embodiment of the present invention, preferably the encapsulating material comprises a polymer, preferably a plasticizer and/or emulsifier.

In another embodiment of the present invention, preferably the encapsulate comprises at least 50wt%, preferably at least 6owt%, preferably at least 70wt%, preferably at least 80wt% of a Schiff's base reaction product of a polyamine with a perfume ketone.

In another embodiment of the present invention, preferably the encapsulating material is essentially free of charged moieties. Preferably the encapsulating material is a non-ionic polymer. This ensures that any coacervation and/or charge interaction between the encapsulating material with any amine and/or ketone moiety present in the hydrophobic perfume-polymer complex_ perfume is avoided. Such interactions are undesirable and tend to form a gel, and impede the dissolution, perfume release and performance profiles of the encapsulate.

In addition, the encapsulate has excellent flowability profile.

### SUMMARY OF THE INVENTION

The present invention provides an encapsulate, a laundry detergent composition comprising an encapsulate and a process for preparing an encapsulate as defined by the claims.

### DETAILED DESCRIPTION OF THE INVENTION

### A process for preparing an encapsulate,

The process comprises the steps of: (i) emulsifying a hydrophobic perfume-polymer complex in water to form an oil in water emulsion; (ii) at least partially dissolving an encapsulating material in water; (iii) removing at least some water from the oil in water emulsion comprising the hydrophobic perfume-polymer complex and the encapsulating material to form an encapsulate. Process steps (i), (ii) and (iii) are described in more detail below.

Step (ii), can be performed prior to or after step (i). In this manner the encapsulating material can be at least partially dissolved in water before or after the hydrophobic perfume-polymer complex is emulsified in water.

Preferably, step (iii) is carried out as soon as possible upon completion of step (i); i.e. the emulsion is dried as quickly as possible upon formation of the oil in water emulsion. It may be highly preferred for step (ii) to be carried out prior to step (i).

Preferably, the process comprises the step of contacting a plasticiser with water. The plasticizer is described in more detail below. The plasticizer can be used to plasticize the encapsulating material. The plasticizer can be contacted to the encapsulating material prior to being contacted with water. For example, the plasticizer and the encapsulating material can be admixed together to form a pre-mix prior to them being contacted with water. Alternatively, the encapsulating material and plasticizer can be dosed separately with the water.

Preferably, the process comprises the step of contacting an emulsifier with water. The emulsifier is described in more detail below. The emulsifier aids the emulsification of the hydrophobic perfume-polymer complex in water. The emulsifier can be contacted with the hydrophobic perfume-polymer complex prior to being contacted with water. For example the emulsifier and the hydrophobic perfume-polymer complex can be admixed together to form a pre-mix prior to them being contacted with water. Alternatively, the hydrophobic perfume-polymer complex and emulsifier can be dosed separately with the water.

### Step (i)

In step (i), the hydrophobic perfume-polymer complex is emulsified in water to form an oil in water emulsion. It may be preferred for the hydrophobic perfume-polymer complex to be formed in-situ during step (i); i.e. the perfume and the polymer are dosed separately to the water and complex together to form the hydrophobic perfume-polymer complex during step (i). It may be preferred for an emulsifier to be present during step (i). Preferably the encapsulating material is present in step (i). Preferably the plasticizer is present in step (i).

Step (i) can be achieved by subjecting the hydrophobic perfume-polymer complex and water to high shear and/or high temperatures, such as using a homogenizers, sonolators, in-line mixers, and at temperatures of from 55°C to 65°C.

### Step (ii)

In step (ii), the encapsulating material is at least partially dissolved in water. Preferably, the encapsulating material is essentially completely dissolved in water. Without wishing to be bound by theory, the Inventors believe that this partial dissolution step enables the encapsulating material to form a film around the hydrophobic perfume-polymer complex. This in turn enables relatively low levels of encapsulating material to be used to adequately encapsulate the perfume, compared to the hot melt agglomeration processes of the prior art, such as those described in WO00/02986.

Step (ii) is preferably carried out at a temperature of from above 90°C to 130°C. However, if the encapsulating material has been subjected to water at elevated temperatures prior to step (ii) (such as a prior jet cooking process step), then step (ii) can be carried out at lower temperatures, such as from 50°C to 90°C. This is especially preferred when the encapsulating material is a starch or derivative thereof. Alternatively, step (ii) can be carried out at ambient conditions. This can be preferred when the encapsulating material is polyvinyl alcohol.

### Step (iii)

In step (iii), at least some water is removed from the oil in water emulsion comprising the hydrophobic perfume-polymer complex and the encapsulating material to form an encapsulate. Step (iii) can be carried out in any suitable drying apparatus, such as a fluid bed dryer, spray-dryer or the like. Preferably, step (iii) is a spray-drying step.

Step (iii) is preferably carried out in a spray-dryer, having an in-let air temperature of from 150°C to 220°C, and/or an out-let air temperature of from 70°C to 120°C. Preferably step (iii) is carried out in a co-current spray-dryer. However, counter-current spray-dryers can also be used.

### Oil in water emulsion

The oil in water emulsion preferably comprises from 15wt% to 90wt% water, preferably from 20wt% or from 30wt% or from 40wt% water, and preferably to 80wt%, or to 70wt%, or to 60wt% water.

Preferably the oil in water emulsion comprises from 0.5wt% to 85wt% oil.

The emulsion preferably has a droplet size of from 0.8 micrometers to 150 micrometers.

### Encapsulate

Typically, the encapsulate comprises a hydrophobic perfume-polymer complex and encapsulating material. Typically, the encapsulating material at least partially, preferably substantially completely, encapsulates the hydrophobic perfume-polymer complex. The encapsulate may also comprise an emulsifier and/or a plasticizer and/or viscosity modifier. The hydrophobic perfume-polymer complex, encapsulating material, plasticizer, emulsifier and viscosity modifier are all described in more detail below.

Preferably, the encapsulate has a weight average particle size of from 10 micrometers to 200 micrometers, preferably from 20 micrometers, and preferably to 100 micrometers, or to 50 micrometers. Such encapsulates having this preferred particle size distribution exhibit improved solubility and have good physical strength; having good particle attrition profiles.

Preferably, the encapsulate comprises at least 50wt%, preferably at least 60wt%, or at least 70wt%, or even at least 80wt% hydrophobic perfume-polymer complex.

Typically, the encapsulate comprises from 0wt% to 20wt%, preferably from 3wt% to 10wt% water.

Typically, the encapsulate has a spheroid shape.

### Encapsulating material

Preferably, the encapsulating material does not melt at a temperature of 135°C or less, preferably 150°C or less.

Preferably, the encapsulating material is a polymer, preferably a film-forming polymer.

Preferably, the encapsulating material is non-ionic, especially preferred is a nonionic polymer, most especially preferred is a non-ionic film-forming polymer.

Preferably, the encapsulating material is maltodextin, more preferably having a dextrose equivalent of from four to twenty DE. The method to determine the dextrose equivalent of the encapsulating material is described in more detail below.

Preferably, the encapsulating material is at least partially water soluble, preferably the encapsulating material is substantially completely water soluble. The method to determine the water solubility of the encapsulating material is described in more detail below.

### Method to determine dextrose equivalent

A typical method to determine dextrose equivalent (DE) is BS EN ISO 5377:1994. In this method: typically in step 7.1.4 the time for boiling to commence is 120 seconds; and typically in step 7.1.9 the successive increments of the glucose solution are added at intervals of 10 seconds, and the addition is completed by 60 seconds; and typically in step 7.2.1 the temperature of the bath is 65°C.

### Method to determine water-solubility

Typically, the method to determine water-soluble is detailed as follows:

Dose 10g of the material to be tested with 1000mL of de-ionized water at 60°C in a 2L glass beaker. Stir the mixture for 5 minutes with an Ika T-25 Ultra-Turrax Digital Homogeniser fitted with a 2G Coarse Rotor-Stator Generator set to 12000RPM unloaded speed.

An aliquot of the resulting mixture is taken for vacuum filtration through a Grade 4 Duran sinter disk glass filter funnel.

Discard the first 10mL of filtrate and then collect a subsequent aliquot in a dry, preweighed glass beaker. Weigh then dry the aliquot to constant weight in a ventilated oven set to 105°C. Allow the beaker to cool to ambient temperature and then re-weigh the beaker to determine the amount of residue.

A material is considered soluble if more than 0.05g per 10mL aliquot is recovered from the filtrate. Preferred solubility is when more than 0.06g, or more than 0.07g, or more than 0.08g or even more than 0.09g per 10mL aliquot is recovered from the filtrate.

### Hydrophobic perfume-polymer complex

The hydrophobic perfume-polymer complex is a complex of perfume raw material and polymer. The complex can be an intimate mix, preferably hydrophobic complex. More preferably the perfume and polymer are co-valently bonded, for example to form a Schiff's base.

Suitable polymers include polyalkylimine, such as polyethyleneimine (PEI), or polyvinylamine (PVAm), silicones including derivatized silicones such as aminosilicones, polyacetal, polyacrylate, polyacrylic, polyacrylonitrile, polyamide, polyaryletherketone, polybutadiene, polybutylene, polybutylene terephthalate, polychloroprene, polyethylene, polyethylene terephthalate, polycyclohexylene dimethylene terephthalate, polycarbonate, polychloroprene, polyhydroxyalkanoate, polyketone, polyester, polyethylene, polyetherimide, polyethersulfone, polyethylenechlorinates, polyimide, polyisoprene, polylactic acid, polymethylpentene, polyphenylene oxide, polyphenylene sulphide, polyphthalamide, polypropylene, polystyrene, polysulphone, polyvinyl acetate, polyvinyl chloride, as well as polymers or copolymers based on acrylonitrile-butadiene, cellulose acetate, ethylene-vinyl acetate, ethylene vinyl alcohol, styrene-butadiene, vinyl acetate-ethylene, and mixtures thereof. A highly preferred polymer is a polyethylene imine or a mixture of polyethylene imine and polycarboxylate. A especially preferred polymer is an ion-pair of polyethylene imine and polycarboxylate.

Any perfume raw is suitable. However, especially suitable perfume raw materials include aldehydes and ketones, including unsaturated ketones and enones such as damascene. A highly preferred perfume raw material is delta-damascone.

Preferably, the hydrophobic perfume-polymer complex is a Schiff's base reaction product of a polyamine, such as a polyethyleneimine, with a perfume ketone. Preferred polyamines comprise a primary or secondary amine moiety. Preferably, the perfume-polymer complex has the general structural formula: 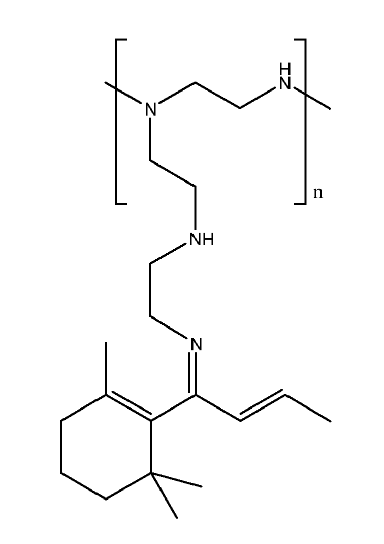 wherein n = greater than 50, preferably greater than 100.

### Plasticiser

Suitable plasticisers include glycerol and/or sorbitol. Preferred plasticisers are non-ionic compounds. Preferred plasticisers have a molecular weight of below 300Da. Suitable plasticisers include but is not limited to corn syrup, sugars, invert sugars, polyols, polyacids, polyesters, polyethers, dimers, disaccharides and low molecular weight (e.g., 300-1800 MW) oligosaccarides of these compounds and mixtures of one or more of these.

Preferred plasticisers include diethylene glycol, sorbitol, sorbitol esters, sucrose, mannitol, maltitol, lactitol, fructose, glucose, high fructose corn syrups, citric acid, ascorbic acid, glycerol, ethylene glycol and xylitol.

### Emulsifier

Preferred emulsifiers are non-ionic compounds. Suitable emulsifiers include polyethoxylates, sorbitan. Preferred nonionic emulsifiers are selected from the group consisting of polyoxyethyleneglycerol monolaurate, polyoxyethyleneglycerol monostearate, polyoxyethylene-20-cetyl stearate, polyoxyethylene-25-cetyl stearate, polyoxyethylene (25)-oxypropylene monostearate, polyoxyethylene-20-sorbitan monopalmitate, poly-oxyethylene-16-tert-octylphenol, polyoxyethylene-20-cetyl ether, polyethylene glycol(1000) monocetyl ether, ethoxylated castor oil, polyoxyethylene sorbitol-lanolin derivatives, polyoxyethylene(25)propylene glycol stearate, polyoxyethylenesorbitol esters, polyoxyethylene-20-sorbitan monopalmitate, polyoxyethylene-16-tert-octylphenol, polyoxyethylene-20-cetyl ether and mixtures thereof.

### Viscosity modifier

Optionally, it may be preferred for a viscosity modifier to be used to control the viscosity of the emulsion. This may help improve the stability of the emulsion. Suitable viscosity modifiers include cellulosic polymers such as carboxymethyl cellulose. Suitable viscosity modifiers also include water-soluble gums and water-soluble synthetic polymers.

A preferred viscosity emulsifier is a water-soluble gum, preferably present in the emulsion at a level of from 0wt% to 10wt% of the emulsion. Preferred water-soluble gums are selected from the group consisting of agar, alginates, carboxymethyl cellulose, carrageenan, gellan, guar gum, gum Arabic, locust bean, pectin and xanthan.

A preferred viscosity emulsifier is a water soluble synthetic polymer, preferably present in the emulsion at a level of from 0wt% to 30wt% of the emulsion. Preferred water-soluble synthetic polymers are polyvinyl alcohols or derivative thereof.

### Laundry detergent composition

The laundry detergent composition comprises the above described encapsulate.

### EXAMPLES

### Process for preparing a perfume encapsulate

1. A Schiff's base reaction product is formed by mixing 240g of delta-damascone with 160g of polyethyleneimine (Lupasol WF^{™}) at 60°C for 24 hours to form 400g of perfume-polymer complex and maintain its temperature at 60°C until added to the mixture below in the later step described below.
2. Dissolve 270g of maltodextrin having a dextrose equivalent in the range of from 12 DE to 20 DE and 27g sorbitol in 670ml water at 60°C.
3. Add 20g of C₁₆ alkyl ethoxylate having an average degree of ethoxylation of 3 to the solution formed in step 2.
4. Stir the mixture formed in step 3 with an Ika T-25 Ultra-Turrax Digital Homogeniser fitted with a 2G Coarse Rotor-Stator Generator set to between 12500 RPM unloaded speed.
5. While still mixing with the Ika T-25 Ultra-Turrax Digital Homogeniser, add 400g of the perfume-polymer complex formed in step 1 to the mixture formed in step 4 at a rate of 200g per minute and continue mixing for 5 minutes after all of the perfume-polymer complex has been added to complete emulsion formation (oil in water emulsion).
6. The resulting emulsion formed in step 5 is spray-dried without delay using a using a Mini Mobile Niro Spray Drier having a rotary atomisation and using an inlet air temperature of 200°C and an outlet air temperature of 95°C to form a perfume encapsulate.

### Perfume encapsulate

The perfume encapsulate prepared by the process described above has a composition of: 5.0wt% water, 53.0wt% perfume-polymer complex (31.8wt% delta-damascone, 21.2wt% Lupasol WF^{™}), 35.8wt% maltodextrin having a dextrose equivalent in the range of from 12 DE to 20 DE, 3.6wt% sorbitan, 2.6wt% C₁₆ alkyl ethoxylate having an average degree of ethoxylation of 3.

The perfume encapsulate prepared by the process described above has a weight average particle size of 50 micrometers.

### Laundry Detergent Composition comprising the perfume encapsulate

A solid laundry detergent composition comprises: 1wt% perfume encapsulate described above, 10wt% detersive surfactant, 10wt% sodium carbonate, 0.3wt% chelant, 0.2wt% brightener, 0.5wt% perfume (and moisture, sodium sulphate and miscellaneous to 100wt%).

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

1. A process for preparing an encapsulate, the process comprises the steps of:
(i) emulsifying a hydrophobic perfume-polymer complex in water to form an oil in water emulsion;
(ii) at least partially dissolving an encapsulating material in water;
(iii) removing at least some water from the oil in water emulsion comprising the hydrophobic perfume-polymer complex and the encapsulating material to form an encapsulate.

2. A process according to claim 1, wherein the encapsulate has a weight average particle size of from 10 microns to 200 microns.

3. A process according to any preceding claim wherein the encapsulating material does not melt at a temperature of 135°C or less, preferably 150°C or less.

4. A process according to any preceding claim, wherein the encapsulating material is a polymer.

5. A process according to any preceding claim, wherein the encapsulating material is a film-forming polymer.

6. A process according to any preceding claim, wherein the encapsulating material is non-ionic.

7. A process according to any preceding claim, wherein the encapsulating material is maltodextin.

8. A process according to any preceding claim, wherein the encapsulating material is maltodextrin having a dextrose equivalent of from four to twenty DE.

9. A process according to any preceding claim, wherein the encapsulate comprises at least 50wt% hydrophobic perfume-polymer complex.

10. A process according to any preceding claim, wherein the hydrophobic perfume-polymer complex is a Schiff's base reaction product of a polyamine with a perfume ketone.

11. A process according to any preceding claim, wherein the process comprises the step of contacting a plasticiser with water.

12. A process according to any preceding claim, wherein the process comprises the step of contacting an emulsifier with water.

13. A process for preparing a laundry detergent composition, the process comprises the process according to any preceding claim.

14. An encapsulate obtained by the process according to any of claims 1-12.

15. A laundry detergent composition comprising an encapsulate according to claim 14.
